# EUROPEAN PATENT SPECIFICATION

(11) **EP 4 426 202 B1**
(45) Date of publication and mention of the grant of the patent: **15.04.2026**
(21) Application number: 22822746.8
(22) Date of filing: 04.11.2022
(51) Int. Cl.: A61B 8/08, A61B 8/00

(54) **SYSTEMS AND METHODS FOR ARTIFICIAL INTELLIGENCE ENABLED ULTRASOUND CORRELATION**
SYSTEME UND VERFAHREN FÜR DURCH KÜNSTLICHE INTELLIGENZ AKTIVIERTE ULTRASCHALLKORRELATION
SYSTÈMES ET PROCÉDÉS DE CORRÉLATION ULTRASONORE ACTIVÉE PAR INTELLIGENCE ARTIFICIELLE

(30) Priority: 05.11.2021 US 202163276497 P
(43) Date of publication of application: 11.09.2024
(73) Proprietor: Bard Access Systems, Inc., Salt Lake City, UT 84116 (US)
(72) Inventor: SOWARDS, Steffan, Salt Lake City, UT 84124 (US); MCLAUGHLIN, William, Robert, Bountiful, UT 84010 (US); MISENER, Anthony, K., Bountiful, UT 84010 (US)
(74) Representative: Hoffmann Eitle
(86) International application number: PCT/US2022/049042
(87) International publication number: WO 2023/081414

(56) References cited:
- US-A1- 2008 161 687
- US-A1- 2012 078 103
- US-A1- 2015 320 325
- US-A1- 2023 026 537

## Description

### BACKGROUND

Ultrasound imaging is a widely accepted tool for guiding interventional instruments such as needles to targets such as blood vessels or organs in the human body. In order to successfully guide, for example, a needle to a blood vessel using ultrasound imaging, the needle is monitored in real-time both immediately before and after a percutaneous puncture in order to enable a clinician to determine the distance and the orientation of the needle to the blood vessel and ensure successful access thereto. However, through inadvertent movement of an ultrasound probe during the ultrasound imaging, the clinician can lose both the blood vessel and the needle, which can be difficult and time consuming to find again. In addition, it is often easier to monitor the distance and orientation of the needle immediately before the percutaneous puncture with a needle plane including the needle perpendicular to an image plane of the ultrasound probe. And it is often easier to monitor the distance and orientation of the needle immediately after the percutaneous puncture with the needle plane parallel to the image plane. As with inadvertently moving the ultrasound probe, the clinician can lose both the blood vessel and the needle when adjusting the image plane before and after the percutaneous puncture, which can be difficult and time consuming to find again. What is needed are ultrasound imaging systems and methods thereof that can dynamically adjust the image plane to facilitate guiding interventional instruments to targets in at least the human body.

Doppler ultrasound is a noninvasive approach to estimating the blood flow through your blood vessels by bouncing high-frequency sound waves (ultrasound) off circulating red blood cells. A doppler ultrasound can estimate how fast blood flows by measuring the rate of change in its pitch (frequency). Doppler ultrasound may be performed as an alternative to more-invasive procedures, such as angiography, which involves injecting dye into the blood vessels so that they show up clearly on X-ray images. Doppler ultrasound may help diagnose many conditions, including blood clots, poorly functioning valves in your leg veins, which can cause blood or other fluids to pool in your legs (venous insufficiency), heart valve defects and congenital heart disease, a blocked artery (arterial occlusion), decreased blood circulation into your legs (peripheral artery disease), bulging arteries (aneurysms), and narrowing of an artery, such as in your neck (carotid artery stenosis). Doppler ultrasound may also detect a direction of blood flow within a blood vesse The prior art document US 2023/026537 A1 discloses an ultrasound guidance system which acquires a first echo image in which a target tissue is displayed on a central axis, acquires a second echo image obtained by scanning a cross section different from the first echo image at a reference position of the ultrasonic probe from which the first echo image was acquired, and compares a central axis of the second echo image with a position of the target tissue in the second echo image.

### SUMMARY

Disclosed herein is an ultrasound imaging system comprising an ultrasound probe including an array of ultrasonic transducers configured to emit generated ultrasound signals into a patient, receive reflected ultrasound signals from the patient, and convert the reflected ultrasound signals into corresponding electrical signals of the ultrasound signals for processing into ultrasound images and a console configured to communicate with the ultrasound probe, the console including one or more processors and a non-transitory computer-readable medium having stored thereon logic, when executed by the one or more processors, causes operations. The operations include capturing a first ultrasound image of a target insertion area of a patient at a first time, capturing a second ultrasound image of the target insertion area at a second time, generating and causing rendering of a notification indicating results of a comparison of the first and second ultrasound images. According to the claimed subject matter, the comparison determines whether the first ultrasound image matches or substantially matches the second ultrasound image such that the position of the ultrasound probe when the second ultrasound image was captured matches or substantially matches the position of the ultrasound probe when the first ultrasound image was captured. In some embodiments, the ultrasound probe includes a piezoelectric array of ultrasonic transducers. In other embodiments, the ultrasound probe includes a micro-electro-mechanical-systems (MEMS) acoustic emission (AE) sensors. In some embodiments, the ultrasound probe includes piezoelectric MEMS (piezo-MEMS) sensors.

In some embodiments, the first time is prior to a sterilization procedure being performed to sterilize an environment surrounding the target insertion area and the second time is subsequent to the sterilization procedure. In some embodiments, the comparison view display includes the first ultrasound image and the second ultrasound image positioned in a horizontal arrangement. In some embodiments, the comparison view display includes the first ultrasound image and the second ultrasound image positioned in a vertical arrangement.

In some embodiments, the comparison view display includes either (i) the first ultrasound image overlaid on the second ultrasound image, or (ii) the second ultrasound image overlaid on the first ultrasound image. In some embodiments, the operations further include: identifying one or more vessels in the first ultrasound image and the second ultrasound image, and providing a visual indication of the one or more vessels in the comparison view display. In some embodiments, the operations further include: determining, through application of a trained machine learning model, whether the second ultrasound image corresponds to the second ultrasound image by at least a threshold amount, and providing a visual indication of a result of applying the trained machine learning model. In some embodiments, the trained machine learning model includes a convolutional neural network.

Also disclosed herein is a method of providing the ultrasound imaging system discussed above and providing instructions to cause performance of the operations also discussed above. Additionally, disclosed herein is a non-transitory, computer-readable medium having logic stored thereon that, when executed by a processor causes performance of the operations discussed above.

These and other features of the concepts provided herein will become more apparent to those of skill in the art in view of the accompanying drawings and following description, which describe particular embodiments of such concepts in greater detail.

### BRIEF DESCRIPTION OF THE DRAWINGS

Embodiments of the disclosure are illustrated by way of example and not by way of limitation in the figures of the accompanying drawings, in which like references indicate similar elements and in which:
FIG. 1 illustrates an ultrasound imaging system and a patient in accordance with some embodiments;
FIG. 2 illustrates a block diagram of a console of the ultrasound imaging system of FIG. 1 in accordance with some embodiments;
FIG. 3A illustrates the ultrasound probe 106 of the ultrasound imaging system 100 imaging a blood vessel of the patient *P* in an unsterile environment 300 prior to accessing the blood vessel in accordance with some embodiments;
FIG. 3B illustrates an ultrasound image of the blood vessel of FIG. 3A on a display screen of the ultrasound imaging system in accordance with some embodiments;
FIG. 4A illustrates the ultrasound probe of the ultrasound imaging system imaging a blood vessel of the patient *P* in a sterile environment prior to accessing and/or while accessing the blood vessel in accordance with some embodiments;
FIG. 4B illustrates an ultrasound image of the blood vessel of FIG. 4A on a display screen of the ultrasound imaging system in accordance with some embodiments;
FIG. 5A illustrates a first display screen including a comparison a pre-scan ultrasound image and a live scan image rendered on the display screen of the ultrasound imaging system of FIG. 1 in accordance with some embodiments;
FIG. 5B illustrates the first display screen of FIG. 5A including reference lines extending vertically in accordance with some embodiments;
FIG. 5C illustrates a second display screen including a comparison a pre-scan ultrasound image and a live scan image rendered on the display screen of the ultrasound imaging system of FIG. 1 in accordance with some embodiments; and
FIG. 6 provides a flowchart illustrating an exemplary method of capturing first ultrasound image at a first time of a target insertion area of a patient *P* and a second ultrasound image at a second, subsequent time following sterilization of the target insertion area, performing a comparison of the images to determine whether the placement of the ultrasound probe following sterilization is in the proper location and orientation in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, ultrasound imaging systems and methods thereof are needed that can dynamically adjust the image plane to facilitate guiding interventional instruments to targets in at least the human body. Disclosed herein are dynamically adjusting ultrasound imaging systems and methods thereof.

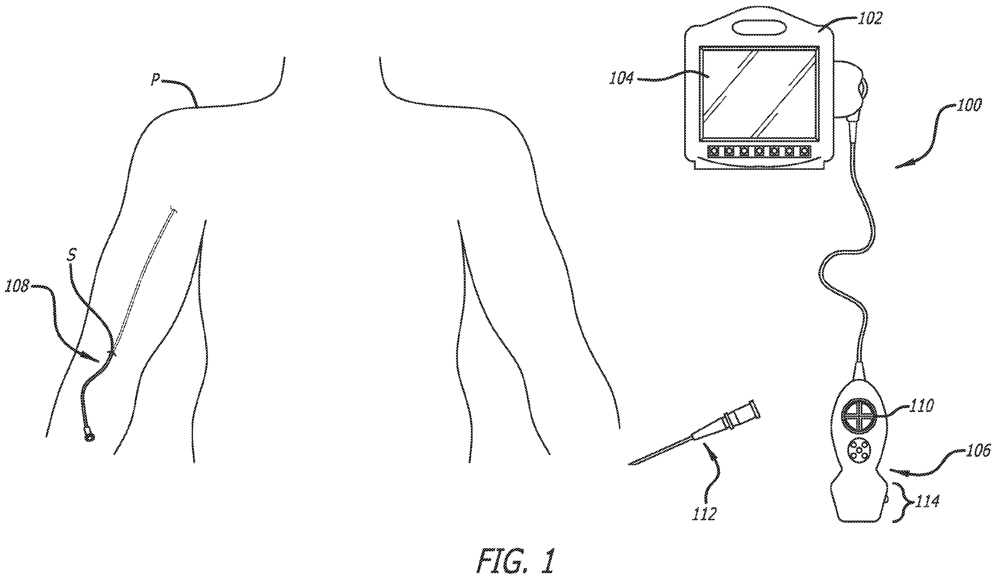

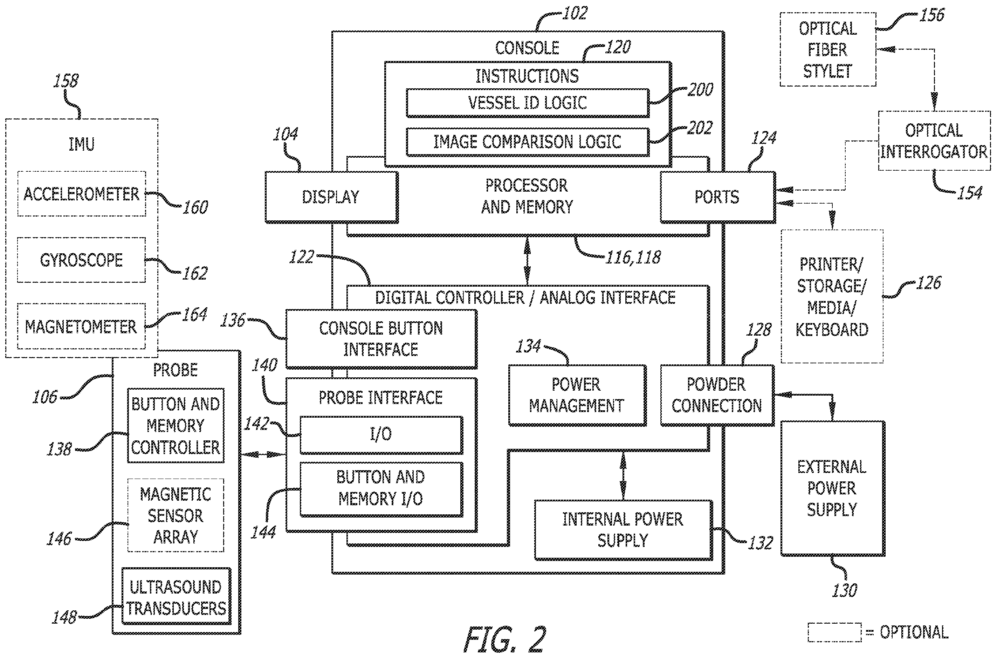

Referring now to FIG. 1, an ultrasound imaging system 100, a needle 112, and a patient P is shown in accordance with some embodiments. FIG. 2 illustrates a block diagram of the ultrasound imaging system 100 in accordance with some embodiments. The discussion below may be made with reference to both FIGS. 1-2. As shown, the ultrasound imaging system 100 includes a console 102, the display screen 104, and the ultrasound probe 106. The ultrasound imaging system 100 is useful for imaging a target such as a blood vessel or an organ within a body of the patient *P* prior to a percutaneous puncture with the needle 112 for inserting the needle 112 or another medical device into the target and accessing the target as well as imaging a target during the insertion process to provide confirmation of the needle 112. Indeed, the ultrasound imaging system 100 is shown in FIG. 1 in a general relationship to the patient *P* during a ultrasound-based medical procedure to place a catheter 108 into the vasculature of the patient *P* through a skin insertion site *S* created by a percutaneous puncture with the needle 112. It should be appreciated that the ultrasound imaging system 100 can be useful in a variety of ultrasound-based medical procedures other than catheterization. For example, the percutaneous puncture with the needle 112 can be performed to biopsy tissue of an organ of the patient P.

The console 102 houses a variety of components of the ultrasound imaging system 100, and it is appreciated the console 102 can take any of a variety of forms. A processor 116 and memory 118 such as random-access memory ("RAM") or non-volatile memory (e.g., electrically erasable programmable read-only memory ("EEPROM")) are included in the console 102 for controlling functions of the ultrasound imaging system 100. The processor may execute various logic operations or algorithms during operation of the ultrasound imaging system 100 in accordance with executable logic ("instructions") 120 stored in the memory 118 for execution by the processor 116. For example, the console 102 is configured to instantiate by way of the logic 120 one or more processes for dynamically adjusting a distance of activated ultrasonic transducers 149 from a predefined target (e.g., blood vessel) or area, an orientation of the activated ultrasonic transducers 149 to the predefined target or area, or both the distance and the orientation of the activated ultrasonic transducers 149 with respect to the predefined target or area, as well as process electrical signals from the ultrasound probe 106 into ultrasound images. Dynamically adjusting the activated ultrasonic transducers 149 uses ultrasound imaging data, magnetic-field data, shape-sensing data, or a combination thereof received by the console 102 for activating certain ultrasonic transducers of a 2-D array of the ultrasonic transducers 148 or moving those already activated in a linear array of the ultrasonic transducers 148. A digital controller/analog interface 122 is also included with the console 102 and is in communication with both the processor 116 and other system components to govern interfacing between the ultrasound probe 106 and other system components set forth herein.

The ultrasound imaging system 100 further includes ports 124 for connection with additional components such as optional components 126 including a printer, storage media, keyboard, etc. The ports 124 can be universal serial bus ("USB") ports, though other types of ports can be used for this connection or any other connections shown or described herein. A power connection 128 is included with the console 102 to enable operable connection to an external power supply 130. An internal power supply 132 (e.g., a battery) can also be employed either with or exclusive of the external power supply 130. Power management circuitry 134 is included with the digital controller/analog interface 122 of the console 102 to regulate power use and distribution.

The display screen 104 is integrated into the console 102 to provide a GUI and display information for a clinician during such as one-or-more ultrasound images of the target or the patient *P* attained by the ultrasound probe 106. In addition, the ultrasound imaging system 100 enables the distance and orientation of a magnetized medical device such as the needle 112 to be superimposed in real-time atop an ultrasound image of the target, thus enabling a clinician to accurately guide the magnetized medical device to the intended target. Notwithstanding the foregoing, the display screen 104 can alternatively be separate from the console 102 and communicatively coupled thereto. A console button interface 136 and control buttons 110 (*see* FIG. 1) included on the ultrasound probe 106 can be used to immediately call up a desired mode to the display screen 104 by the clinician for assistance in an ultrasound-based medical procedure. In some embodiments, the display screen 104 is an LCD device.

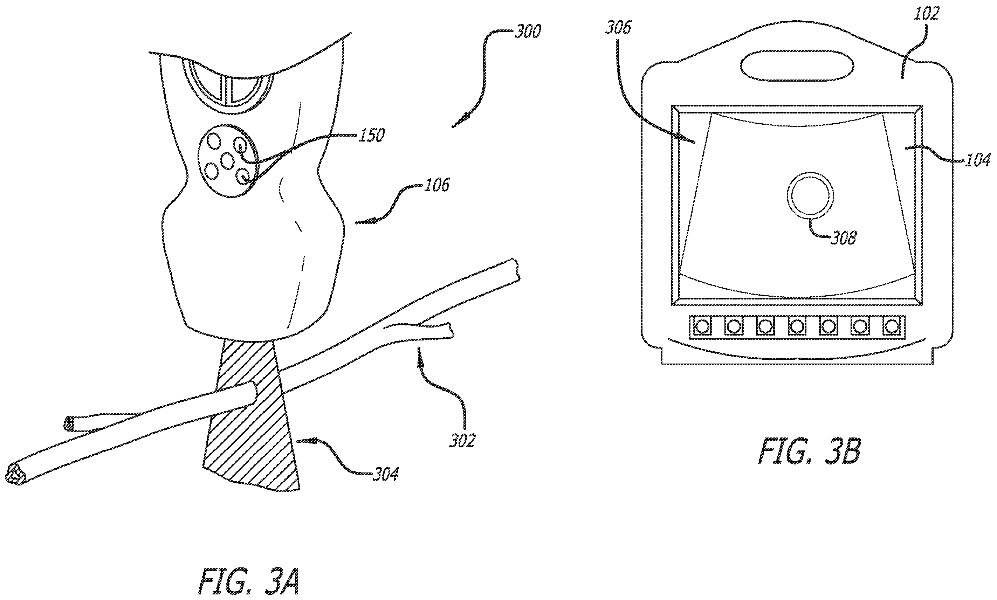

The ultrasound probe 106 is employed in connection with ultrasound-based visualization of a target such as a blood vessel (*see* FIG. 3A) in preparation for inserting the needle 112 or another medical device into the target. Such visualization gives real-time ultrasound guidance and assists in reducing complications typically associated with such insertion, including inadvertent arterial puncture, hematoma, pneumothorax, etc. As described in more detail below, the ultrasound probe 106 is configured to provide to the console 102 electrical signals corresponding to both the ultrasound imaging data, the magnetic-field data, the shape-sensing data, or a combination thereof for the real-time ultrasound guidance.

Optionally, a stand-alone optical interrogator 154 can be communicatively coupled to the console 102 by way of one of the ports 124. Alternatively, the console 102 can include an integrated optical interrogator integrated into the console 102. Such an optical interrogator is configured to emit input optical signals into a companion optical-fiber stylet 156 for shape sensing with the ultrasound imaging system 100, which optical-fiber stylet 156, in turn, is configured to be inserted into a lumen of a medical device such as the needle 112 and convey the input optical signals from the optical interrogator 154 to a number of FBG sensors along a length of the optical-fiber stylet 156. The optical interrogator 154 is also configured to receive reflected optical signals conveyed by the optical-fiber stylet 156 reflected from the number of FBG sensors, the reflected optical signals indicative of a shape of the optical-fiber stylet 156. The optical interrogator 154 is also configured to convert the reflected optical signals into corresponding electrical signals for processing by the console 102 into distance and orientation information with respect to the target for dynamically adjusting a distance of the activated ultrasonic transducers 149, an orientation of the activated ultrasonic transducers 149, or both the distance and the orientation of the activated ultrasonic transducers 149 with respect to the target or the medical device when it is brought into proximity of the target. For example, the distance and orientation of the activated ultrasonic transducers 149 can be adjusted with respect to a blood vessel as the target. Indeed, an image plane can be established by the activated ultrasonic transducers 149 being perpendicular or parallel to the blood vessel in accordance with an orientation of the blood vessel.

FIG. 2 shows that the ultrasound probe 106 further includes a button and memory controller 138 for governing button and ultrasound probe 106 operation. The button and memory controller 138 can include non-volatile memory (e.g., EEPROM). The button and memory controller 138 is in operable communication with a probe interface 140 of the console 102, which includes an input/output ("I/O") component 142 for interfacing with the ultrasonic transducers 148 and a button and memory I/O component 144 for interfacing with the button and memory controller 138.

Also as seen in FIG. 2, the ultrasound probe 106 can include a magnetic-sensor array 146 for detecting a magnetized medical device such as the needle 112 during ultrasound-based medical procedures. The magnetic-sensor array 146 includes a number of magnetic sensors 150 embedded within or included on a housing of the ultrasound probe 106. The magnetic sensors 150 are configured to detect a magnetic field or a disturbance in a magnetic field as magnetic signals associated with the magnetized medical device when it is in proximity to the magnetic-sensor array 146. The magnetic sensors 150 are also configured to convert the magnetic signals from the magnetized medical device (e.g., the needle 112) into electrical signals for the console 102 to process into distance and orientation information for the magnetized medical device with respect to the predefined target, as well as for display of an iconographic representation of the magnetized medical device on the display screen 104. Thus, the magnetic-sensor array 146 enables the ultrasound imaging system 100 to track the needle 112 or the like.

Though configured here as magnetic sensors, it is appreciated that the magnetic sensors 150 can be sensors of other types and configurations. Also, though they are described herein as included with the ultrasound probe 106, the magnetic sensors 150 of the magnetic-sensor array 146 can be included in a component separate from the ultrasound probe 106 such as a sleeve into which the ultrasound probe 106 is inserted or even a separate handheld device. The magnetic sensors 150 can be disposed in an annular configuration about the probe head 114 of the ultrasound probe 106, though it is appreciated that the magnetic sensors 150 can be arranged in other configurations, such as in an arched, planar, or semi-circular arrangement.

Each magnetic sensor of the magnetic sensors 150 includes three orthogonal sensor coils for enabling detection of a magnetic field in three spatial dimensions. Such 3-dimensional ("3-D") magnetic sensors can be purchased, for example, from Honeywell Sensing and Control of Morristown, NJ. Further, the magnetic sensors 150 are configured as Hall-effect sensors, though other types of magnetic sensors could be employed. Further, instead of 3-D sensors, a plurality of 1-dimensional ("1-D") magnetic sensors can be included and arranged as desired to achieve 1-, 2-, or 3-D detection capability.

As shown in FIG. 2, the ultrasound probe 106 can further include an inertial measurement unit ("IMU") 158 or any one or more components thereof for inertial measurement selected from an accelerometer 160, a gyroscope 162, and a magnetometer 164 configured to provide positional-tracking data of the ultrasound probe 106 to the console 102 for stabilization of an image plane. The processor 116 is further configured to execute the logic 120 for processing the positional-tracking data for adjusting the distance of the activated ultrasonic transducers 149 from the target, the orientation of the activated ultrasonic transducers 149 to the target, or both the distance and the orientation of the activated ultrasonic transducers 149 with respect to the target to maintain the distance and the orientation of the activated ultrasonic transducers 149 with respect to the target when the ultrasound probe 106 is inadvertently moved with respect to the target.

It is appreciated that a medical device of a magnetizable material enables the medical device (e.g., the needle 112) to be magnetized by a magnetizer, if not already magnetized, and tracked by the ultrasound imaging system 100 when the magnetized medical device is brought into proximity of the magnetic sensors 150 of the magnetic-sensor array 146 or inserted into the body of the patient P during an ultrasound-based medical procedure. Such magnetic-based tracking of the magnetized medical device assists the clinician in placing a distal tip thereof in a desired location, such as in a lumen of a blood vessel, by superimposing a simulated needle image representing the real-time distance and orientation of the needle 112 over an ultrasound image of the body of the patient P being accessed by the magnetized medical device. Such a medical device can be stainless steel such as SS 304 stainless steel; however, other suitable needle materials that are capable of being magnetized can be employed. So configured, the needle 112 or the like can produce a magnetic field or create a magnetic disturbance in a magnetic field detectable as magnetic signals by the magnetic-sensor array 146 of the ultrasound probe 106 so as to enable the distance and orientation of the magnetized medical device to be tracked by the ultrasound imaging system 100 for dynamically adjusting the distance of the activated ultrasonic transducers 149, an orientation of the activated ultrasonic transducers 149, or both the distance and the orientation of the activated ultrasonic transducers 149 with respect to the magnetized medical device. In some embodiments, the needle 112 can be tracked using the teachings of one or more patents of U.S. Patent Nos.: 5,775,322; 5,879,297; 6,129,668; 6,216,028; and 6,263,230.

In some embodiments, the distance and orientation information determined by the ultrasound imaging system 100, together with an entire length of the magnetized medical device, as known by or input into the ultrasound imaging system 100, enables the ultrasound imaging system 100 to accurately determine the distance and orientation of the entire length of the magnetized medical device, including a distal tip thereof, with respect to the magnetic-sensor array 146. This, in turn, enables the ultrasound imaging system 100 to superimpose an image of the needle 112 on an ultrasound image produced by the ultrasound beam 152 of the ultrasound probe 106 on the display screen 104. For example, the ultrasound image depicted on the display screen 104 can include depiction of the surface of the skin of the patient P and a subcutaneous blood vessel thereunder to be accessed by the needle 112, as well as a depiction of the magnetized medical device as detected by the ultrasound imaging system 100 and its orientation to the vessel. The ultrasound image corresponds to an image acquired by the ultrasound beam 152 of the ultrasound probe 106. It should be appreciated that only a portion of an entire length of the magnetized medical device is magnetized and, thus, tracked by the ultrasound imaging system 100.

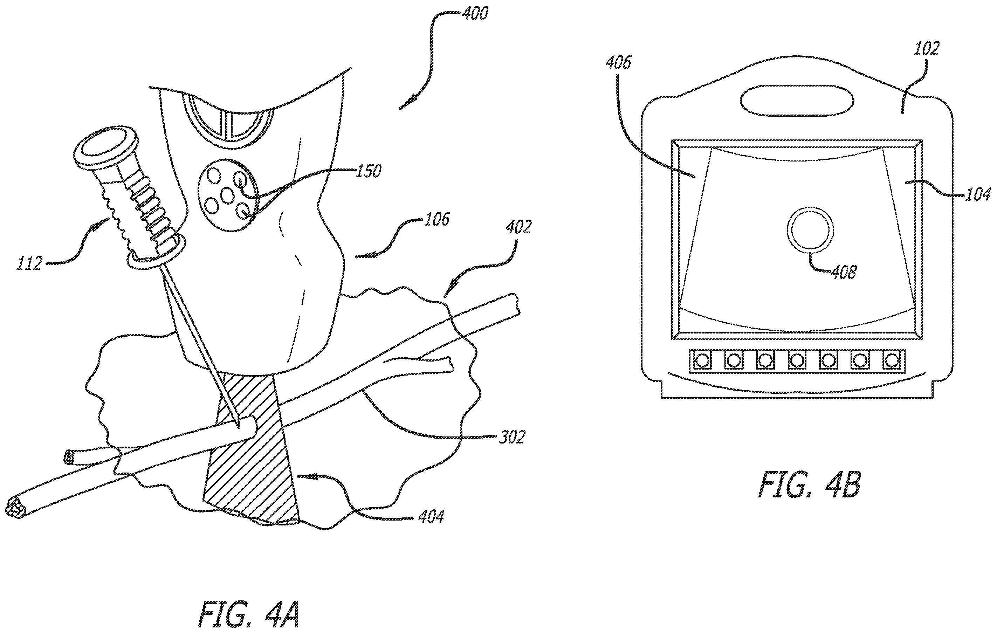

During operation of the ultrasound imaging system 100, the probe head 114 of the ultrasound probe 106 is placed against skin of the patient *P*. An ultrasound beam 152 is produced so as to ultrasonically image a portion of a target such as a blood vessel beneath a surface of the skin of the patient *P.* (*See* FIGS. 3A, 4A.) The ultrasonic image of the blood vessel can be depicted and stabilized on the display screen 104 of the ultrasound imaging system 100 as shown in FIGS. 3B, 4B despite inadvertent movements of the ultrasound probe 106. Note that further details regarding structure and operation of the ultrasound imaging system 100 can be found in U.S. Patent No. 9,456,766, titled "Apparatus for Use with Needle Insertion Guidance System".

FIG. 3A illustrates the ultrasound probe 106 of the ultrasound imaging system 100 imaging a blood vessel of the patient *P* in an unsterile environment 300 prior to accessing the blood vessel in accordance with some embodiments. The imaging performed in FIG. 3A may be referred to as pre-scan imaging. FIG. 3B illustrates an ultrasound image of the blood vessel of FIG. 3A (a "pre-scan image") 306 on a display screen 104 of the ultrasound imaging system 100 in accordance with some embodiments.

The pre-scan image 306 may be obtained at first time that is prior to preparing the patient *P* and the surrounding area for sterilization, where the pre-scan image 306 may be stored in the memory 118 of the console 102. The intended purpose of obtaining the pre-scan image 306 is to allow a clinician to obtain an image of the target vessel 302 using the ultrasound probe 106 without any constraints that may be imposed in order to maintain a sterile environment. As will be discussed below, the pre-scan image may then be used as a reference image to compare to the live scan image taken in a sterile field thereby allowing the clinician to confirm proper placement and orientation of the ultrasound probe 106.

In some embodiments, following operations to obtain, capture, and optionally to store, the pre-scan image, vessel identification logic 200 may be executed by the processor 116 causing performance of operations to identify a visual representation of the target vessel 302, such as the target vessel image 308 of FIG. 3B, within the pre-scan image 306 and/or detect other features of the pre-scan image 306. Other features detected may include those anatomical features typically visualized in an ultrasound image such as blood vessels, bones, muscles, tendons, ligaments, nerves, joints, etc.

The vessel identification logic 200 may be configured, upon execution by the processor 116, to cause performance of operations including computerized, automated analysis of the pre-scan image 306 to identify the target vessel image 308 through machine learning operations (e.g., application of a trained machine learning model). For instance, computerized, automated analysis may include operations comprising object recognition such as object detection methods, where the vessel identification logic 200 parses the pre-scan image 306 to locate a presence of one or more objects (e.g., the target vessel 302) with a bounding box and classify (label) the object within the bounding box. In order to perform such operations, the vessel identification logic 200 may include a machine learning model trained through supervised machine learning using a labeled data set. For example, a labeled data set may include ultrasound images that were previously captured ("historical data") that has also been labeled, e.g., by another trained machine learning model and/or by a subject matter expert. The machine learning model is then trained on the labeled historical data so that upon completion of the training, the machine learning model may detect objects within a new image (e.g., the pre-scan image 306 and a live scan image discussed below with respect to FIGS. 4A-4B), place bounding boxes around the images and classify the images. It is noted that is some embodiments, the classification step may be skipped such that the trained machine learning model is configured to output an image including bounding boxes around detected objects within the image.

In some embodiments, the machine learning model is a convolutional neural network (CNN). As is known, a CNN analyzes an image by sliding a "window" (X x Y set of pixels) throughout the image (e.g., left to right and top to bottom) and utilizes a weighted sum of pixel values of the image to produce a secondary image, which occurs in one convolution layer. This process of sliding the window across the image (or subsequent images) ("convolving") may occur several times based on the number of convolution layers that comprise the CNN.

In some embodiments, the ultrasound probe 106 may be configured for doppler ultrasound such that the blood flow direction of a captured vessel may be determined. Doppler ultrasound images may be advantageous when a clinician is visually comparing a pre-scan image with a live scan image. In some embodiments, systems and methods described herein may not include the vessel identification logic 200 or the image comparison logic 202 (discussed below) and thus, the clinician may not be provided with an automated determination as to whether a live scan image matches (or substantially matches) a pre-scan image. However, systems and methods described herein that do not include the vessel identification logic 200 or the image comparison logic 202 may render the pre-scan and live-scan images on a display enabling the clinician to visually compare the two to determine whether the ultrasound probe 106 is in the correct location. In such embodiments, doppler ultrasound images are advantageous as the indication of blood flow direction provides the clinician with an additional parameter to consider when determining whether the live scan and pre-scan images match.

As used herein, reference to a first image "matching" (or substantially matching) a second image refers to the values of the pixels of the first image being within a threshold of the values of the pixels of the second image. In some embodiments, this may refer a threshold comparison of each pixel of the first image with the corresponding pixel of the second image. In some embodiments, a percentage of the corresponding values are to be within the threshold values in order to be matching (e.g., 75%, 80%, 90%, etc., of the values between corresponding pixels are to be within the threshold). In other embodiments, the first and second images may be broken into corresponding regions (M x N pixels) where the values of the pixels within each region are averaged and each region of the first image is compared to the corresponding region of the second image, where the value of the corresponding regions are to be within a threshold of value of each other. More generally, the term "matching" may refer to a level of correspondence between first and second ultrasound images.

The image comparison logic 202 is configured to, upon execution by the processor 116, cause performance of operations that compare a first image to a second image to determine whether the first image matches (or substantially matches) the second image. For instance, the image comparison logic 202 may be configured to, upon execution by the processor 116, cause performance of operations that compare a live scan image to a pre-scan image to determine whether the live scan image matches the pre-scan image. Based on the operations to compare whether the live scan image matches the pre-scan image, the image comparison logic 202 provides a notification as to whether a matched (or substantial match) occurred. Such a notification may assist a clinician in understanding whether the ultrasound probe 106 is positioned properly, i.e. in a position that matches the positioning when the pre-scan image was captured.

FIG. 4A illustrates the ultrasound probe 106 of the ultrasound imaging system 100 imaging a blood vessel of the patient P in a sterile environment 400 prior to accessing and/or while accessing the blood vessel in accordance with some embodiments. The imaging performed in FIG. 4A may be referred to as live scan imaging. FIG. 4B illustrates an ultrasound image of the blood vessel of FIG. 4A (a "live scan image") on a display screen 104 of the ultrasound imaging system 100 in accordance with some embodiments.

The live scan image 406 may be obtained at second time that is subsequent to creating a sterilized area 402 around an insertion site on the patient *P* (or generally an area on the patient *P*. The live scan image 406 may also be stored in the memory 118 of the console 102. As noted above, systems and methods disclosed herein may include obtaining a pre-scan image 306 with the intended purpose of allowing a clinician to use the pre-scan image 306 as a reference image to compare to the live scan image 406 (which is taken in a sterile field) thereby allowing the clinician to confirm proper placement and orientation of the ultrasound probe 106 during the live scan process, which may correspond to insertion of a medical device such as the needle 112.

In some embodiments, following operations to obtain, capture, and optionally to store, the live scan image 406, the vessel identification logic 200 may be executed by the processor 116 causing performance of operations to identify a visual representation of the target vessel 302, such as the target vessel image 308, within the live scan image 406 and/or detect other features of the live scan image 406. Other features detected may include those anatomical features typically visualized in an ultrasound image such as blood vessels, bones, muscles, tendons, ligaments, nerves, joints, etc.

In some embodiments, the comparison of a pre-scan image and a live scan image may be performed via an automated, computerized method. In some embodiments, such a method may include machine learning techniques (e.g., artificial intelligence). As discussed above, the instructions 120 of the console 102 may include image comparison logic 202 that may be configured to, upon execution by the processor 116, cause performance of operations that compare a first image (e.g., pre-scan image 306) to a second image (e.g., live scan image 406) to determine whether the pre-scan image 306 matches (or substantially matches) the live scan image 406. Such a notification may assist a clinician in understanding whether the ultrasound probe 106 is positioned properly, e.g., in a position that matches the positioning when the pre-scan image was captured).

In some embodiments, some machine learning techniques used to perform the automated, computerized method of comparing the live scan to the pre-scan may include, but the disclosure is not limited or restricted to: keypoint detection and matching; and/or determining the Euclidean distance between image vectors representing the live scan image and the pre-scan image. In some embodiments, a denoising autoencoder may be utilized by the image comparison logic 202 prior to the comparison in order to remove noise.

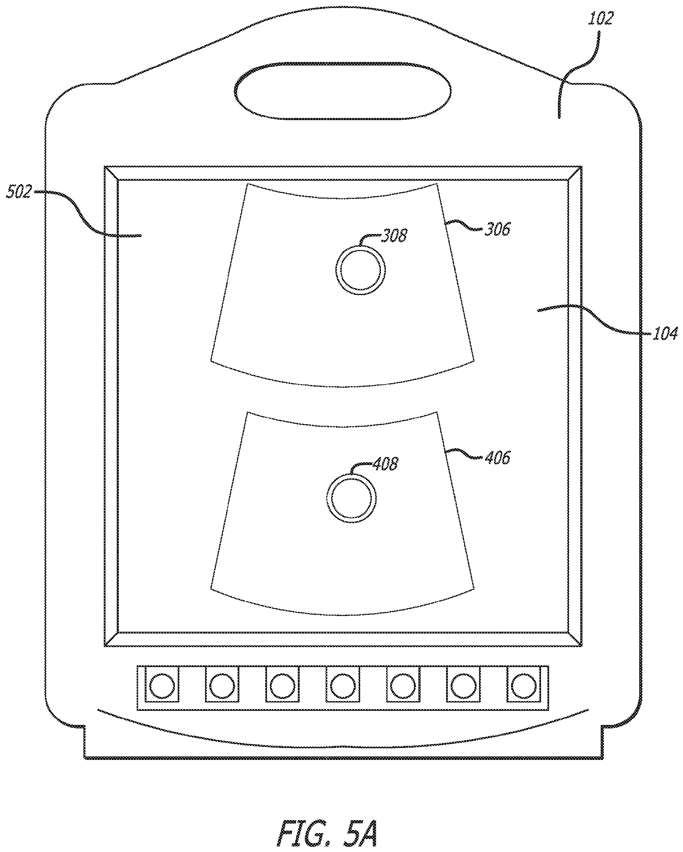

However, other embodiments of systems and methods disclosed herein may not include an automated, computerized method comparing a pre-scan image and a live scan image but instead provide the images to a clinician in a variety of manners that advantageously enable the clinician to determine whether the live scan image matches the pre-scan image thereby allowing the clinician to confirm proper placement and orientation of the ultrasound probe 106 during the live scan process. Examples of visual representations of comparisons of a pre-scan image and a live scan image generated by systems and methods disclosed herein are provided in FIGS. 5A-5B, discussed below.

Referring now to FIG. 5A, an illustration of a first display screen including a comparison a pre-scan ultrasound image and a live scan image rendered on the display screen 104 of the ultrasound imaging system 100 of FIG. 1 is shown in accordance with some embodiments. FIG. 5A illustrates that the instructions 120 may include logic that generates a display 500 and causes rendering of such on the display screen 104 of the console 102. In particular, the display 500 includes both of the pre-scan image 306 and the live scan image 406, which allows a clinician to perform a visual comparison to determine whether the images match (or substantially match) and thus determine whether the positioning and orientation of the ultrasound probe 106 at a current state (e.g., during live scanning process) matches (or substantially matches) the positioning and orientation of the ultrasound probe 106 at the time that the pre-scan image 306 was captured.

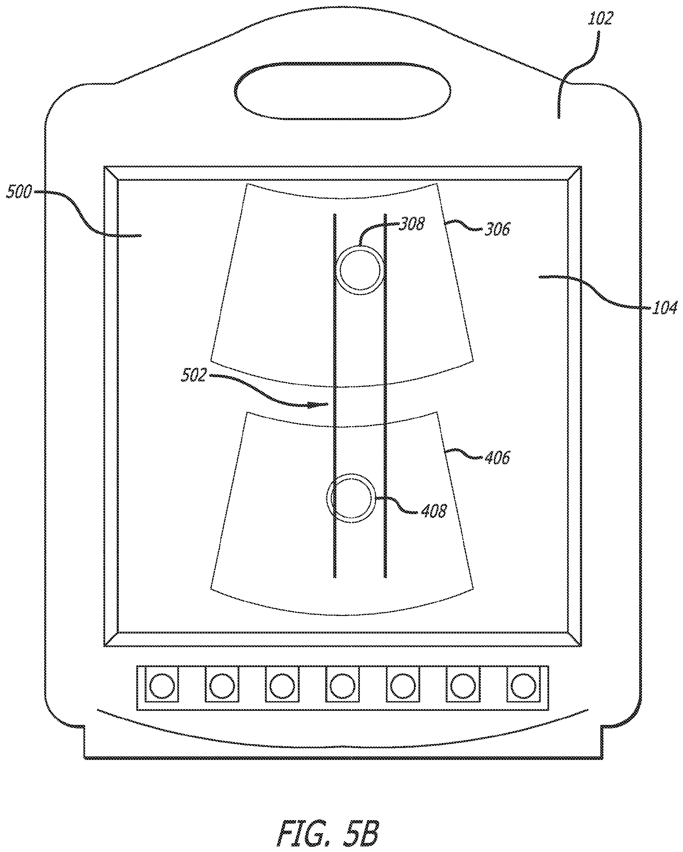

In this embodiment of FIG. 5A, the images are aligned vertically. However, FIG. 5A is not intended to be limiting such that the images may be aligned in other orientations, e.g., horizontally (not shown). Referring to FIG. 5B, an illustration of the first display screen of FIG. 5A is shown including reference lines extending vertically in accordance with some embodiments. The optional visual alignment markers 502 provide a visual indication as to the alignment of the target vessel images 308, 408, which thereby simplifies the job of the clinician to determine whether images 306, 406 match (or substantially match).

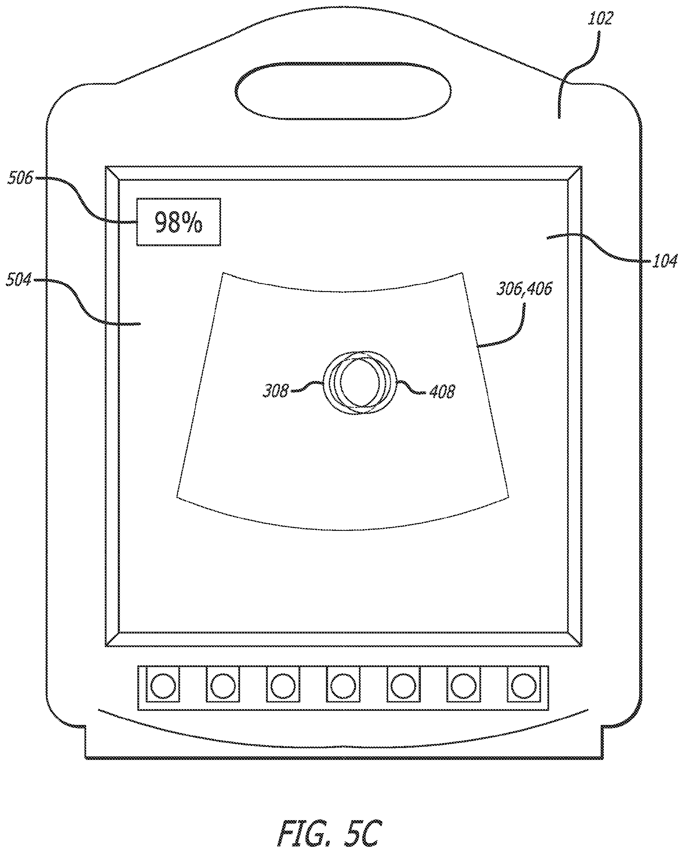

Referring now to FIG. 5C, an illustration of a second display screen including a comparison a pre-scan ultrasound image and a live scan image rendered on the display screen 104 of the ultrasound imaging system 100 of FIG. 1 is shown in accordance with some embodiments. The display 504 rendered on the display screen 104 in FIG. 5C provides an illustration of one image overlaid on the other (e.g., the live scan image 406 as an overlay on the pre-scan image 306 or vice versa). In some embodiments, the opacity of the image used an overlay may be less than 100% (e.g., partial opacity), which may increase the ease of viewing features (e.g., the target vessel images 306, 406) of each of the images. As a result, a clinician may more easily view the location of certain features in one image compared to other corresponding features in the other image. In the sample illustration of FIG. 5C, the clinician may easily see that the two images are very similar (e.g., a match or substantial match) and thus, determine that the current positioning and orientation of the ultrasound probe 106 (corresponding to the live scan image 406) is substantially similar to the positioning and orientation of the ultrasound probe 106 at the time that the pre-scan image 306 was captured.

Additionally, in some embodiments, such as those in which an automated, computerized method for comparing the images is performed, a notification of the result may be caused to be rendered on the display screen 104. As shown, the notification 506 indicates that the two images are a 98% match. This percentage may pertain to a percentage of each pixel within the image that matches (e.g., is within a threshold of each other, such as within a certain number of color or grayscale values) or may be a confidence provided by the machine learning techniques that that the images match (or do not match in some embodiments).

In some embodiments of any of FIGS. 5A-5B, the two images may be displayed in different colors in order to easily distinguish between the images. Additionally, in some embodiments, the ultrasound probe 106 may be configured to perform a doppler ultrasound procedure such that the blood flow direction within vessels is depicted. Such may be advantageous when there are multiple vessels of similar size within the images, whereby the clinician may easily determine corresponding vessel pairs within the two images.

Methods of the foregoing ultrasound imaging systems include methods implemented in the ultrasound imaging systems. For example, a method of the ultrasound imaging system 100 includes a non-transitory CRM (e.g., EEPROM) having the logic 120 stored thereon that causes the ultrasound imaging system 100 to perform a set of operations for ultrasound imaging when the logic 120 is executed by the processor 116 of the console 102. Such a method may generally include activating and capturing operations, processing operations, and displaying operations.

The activating and capturing operations include activating the ultrasonic transducers of the array of the ultrasonic transducers 148 of the ultrasound probe 106 communicatively coupled to the console 102. With the activating operations, the ultrasonic transducers 148 emit generated ultrasound signals into the patient *P*, receive reflected ultrasound signals from the patient *P*, and convert the reflected ultrasound signals into corresponding electrical signals for processing into ultrasound images. The activating operations can include activating an approximately linear subset of the ultrasonic transducers 148 of a 2-D array of the ultrasonic transducers 148. Alternatively, the activating operations can include activating a subset of the ultrasonic transducers 148 up to all the ultrasonic transducers 148 in the movable linear array of the ultrasonic transducers 148. Additionally, capturing operations may include saving the ultrasound images into non-transitory, computer-readable medium such as the memory 118.

The processing operations include processing the corresponding electrical signals of the ultrasound signals including doppler ultrasound signals into the ultrasound images. The processing operations may further include determining a shape of a target blood vessel rendered within the ultrasound image. The determining may also include identifying a length and a width of an elliptical target blood vessel image and further include calculating a parameter related to a difference between the length and the width such as a ratio, for example.

The processing operations may include differentiating a vein image from an artery image within the ultrasound image based on anatomical awareness such as a spatial awareness of a target blood vessel with respect to other blood vessels or anatomical elements. Similarly, the operations may include differentiating a target blood vessel from adjacent blood vessels based on anatomical awareness. In some embodiments, the logic 120 may compare target blood vessel image with one or more ultrasound images stored in memory 118. As a result of the comparison, the logic 120 may determine with a degree of confidence (e.g., a percent probability) that the target blood vessel image is indeed an image of target blood vessel based on anatomical spatial awareness the target blood vessel in relation to adjacent anatomical elements, such as blood vessels, bones, and the like. In some embodiments, the logic 120 may determine a direction of blood flow within the target blood vessel with respect to the ultrasound image of the target blood vessel based at least partially on the anatomical awareness of the target blood vessel.

The processing operations may further include receiving doppler ultrasound data from the ultrasound probe 106 and processing the doppler ultrasound data to determine indicating a direction and/or velocity within the target blood vessel with respect to the ultrasound image plane. The display operations may then render an indicium on the display 104 in combination with the ultrasound image of the target blood vessel where the indicium indicates the direction of blood flow with respect to the target blood vessel image.

As to magnetic signal-related operations, the method can include a converting operation. The converting operation includes converting magnetic signals from a magnetized medical device (e.g., the needle 112) with the magnetic-sensor array 146 of the ultrasound probe 106 into corresponding electrical signals. The processing operations further include processing the corresponding electrical signals of the magnetic signals with the processor 116 into distance and orientation information with respect to the predefined target or area. The displaying operations further include displaying an iconographic representation of the medical device on the display screen 104 (e.g., illustrating an iconographic representation of the needle 112 on the display screen 104 following insertion of the needle 112 into the patient *P*).

The method may further include a number of optical signal-related operations in combination with further processing and displaying operations. The optical signal-related operations include emitting input optical signals, receiving reflected optical signals, and converting the reflected optical signals into corresponding electrical signals of the optical signals by the optical interrogator 154. The optical signal-related operations also include conveying the input optical signals from the optical interrogator 154 to the number of FBG sensors along the length of the optical-fiber stylet 156, as well as conveying the reflected optical signals from the number of FBG sensors back to the optical interrogator 154 with the optical-fiber stylet 156 disposed in a lumen of the medical device. The processing operation further include processing the corresponding electrical signals of the optical signals with the processor 116 into distance and orientation information with respect to the predefined target or area. The displaying operations further include displaying an iconographic representation of a medical device on the display 104.

Additionally, processing operations may include comparing two images captured via the activating and capturing operations. For instance, a first image captured at a first time and a second image captured at a second (subsequent) time may be compared to determine whether the images are matches (or substantial matches). In some embodiments, the first image may be of a target insertion area of a patient *P* prior to any sterilization and the second image may be of the target insertion area following a sterilization process. Thus, the first image may have been captured at a first time, a clinician may have moved the ultrasound probe, sterilized the area and replaced the ultrasound probe at which time the second image was captured. In order to ensure the ultrasound probe was replaced in the proper location, the first and second images are compared to determine whether the images match (or substantially match), which indicates whether the ultrasound probe is image the same location and from the same orientation. As discussed above, the comparison may include operations such as generating visual illustrations of the images (e.g., side-by-side in a horizontal or vertical manner, or overlaid on each other). Alternative embodiments may include an automated, computerized method of comparing the images using, for example, machine learning techniques.

The displaying operations include displaying images on the display 104 communicatively coupled to the console 102 including the ultrasound images. The display operations may further include rendering an indicium on the display in combination with a blood vessel image identifying the blood vessel image as an image of the target blood vessel. Additionally, the displaying operations include causing rendering visual illustrations of the images (e.g., side-by-side in a horizontal or vertical manner, or overlaid on each other) and/or an indication of the result of the automated, computerized method of comparing the images.

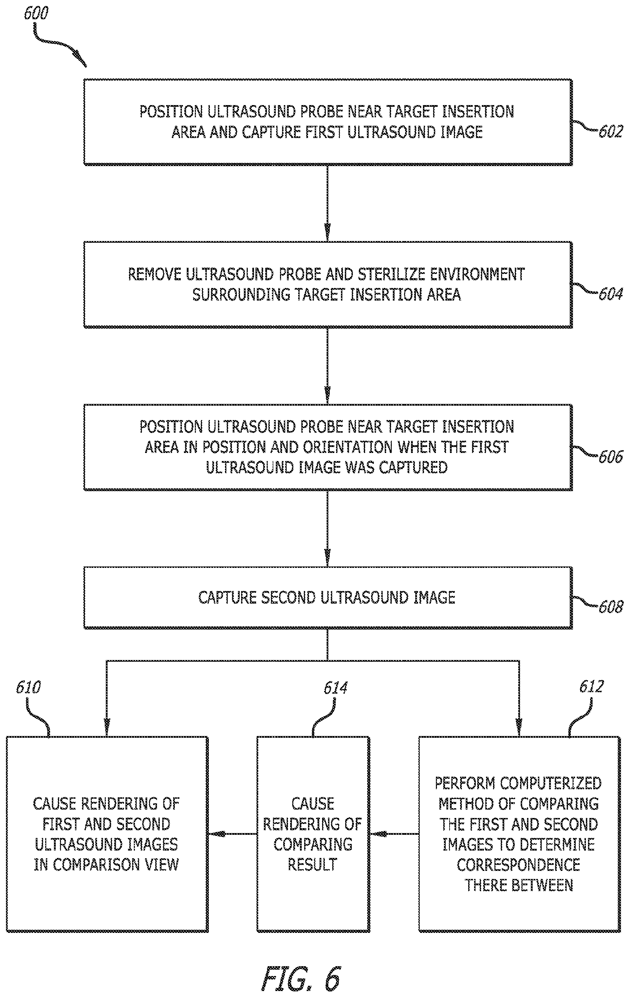

Referring to FIG. 6, a flowchart illustrating an exemplary method of capturing first ultrasound image at a first time of a target insertion area of a patient *P* and a second ultrasound image at a second, subsequent time following sterilization of the target insertion area, performing a comparison of the images to determine whether the placement of the ultrasound probe following sterilization is in the proper location and orientation is shown in accordance with some embodiments. Each block illustrated in FIG. 6 represents an operation of the method 600. It should be understood that not every operation illustrated in FIG. 6 is required. In fact, certain operations may be optional to complete aspects of the method 600. The discussion of the operations of method 600 may be done so with reference to any of the previously described figures. Prior to the initiation of the method 600, it may be assumed that a clinician has access to the ultrasound imaging system 100 of FIG. 1.

The method 600 begins when an ultrasound probe is positioned near a target insert area of a patient *P* and a first ultrasound image is captured (block 602). For instance, as seen in FIG. 3A, the ultrasound probe 106 may be positioned generally above a target vessel, where the target vessel is the intended vessel for which a medical device (e.g., a needle) is to be inserted. As shown in FIG. 3B, the captured ultrasound image may be displayed on a display screen of a console of the ultrasound imaging system 100. The first image may be captured while the environment surrounding the target insertion area has not been sterilized. Thus, the first image may be a "pre-scan" image that is obtained by a clinician of the target insertion area prior to sterilization thereof in order to allow the clinician to determine the positioning and orientation of the ultrasound probe without the restrictions of doing so within a sterile environment (e.g., without the inclusion of draping, sterile gloves, gowns, masks, etc., that may make the process of obtaining positioning and orienting the ultrasound probe difficult).

Following the capture of the first image, the ultrasound probe is removed and the environment surrounding the target insertion area is sterilized (block 604). As noted above, the sterilization process may include positioning of draping, donning of personal protective equipment (PPE) as well as cleaning of medical equipment to be used in the insertion process. Following sterilization, the ultrasound probe is again positioned near the target insertion area in the position and orientation of the ultrasound probe when the first image was captured (block 606). Thus, by initially positioning and orienting the ultrasound probe when capturing the first image, the clinician obtained an idea of the proper (desired) positioning and orientation of the ultrasound probe. A second ultrasound image is then captured with the ultrasound probe positioned and oriented in what the clinician believes to be the same position and orientation as the ultrasound probe when the first image was captured.

The method 600 may continue in either or both of two paths. Following the capture of the second image, the method 600 may include performance of operations that cause the rendering of the first and second ultrasound images in a comparison view (block 610). Examples of comparison views are discussed above and some are illustrated in FIGS. 5A-5C.

Additionally, following the capture of the second image, the method 600 may include performance of operations comprising a computerized method of comparing the first and second images to determine the correspondence therebetween (e.g., whether the first and second images match, or substantially match) (block 612). Operations of embodiments of the computerized methods for comparing the first and second images are discussed above. Subsequently, the result of the method to compare the first and second images may be rendered for viewing by the clinician (block 614). For example, see FIG. 5C.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. An ultrasound imaging system (100), comprising:
an ultrasound probe (106) including an array of ultrasonic transducers (148) configured to emit generated ultrasound signals into a patient (P), receive reflected ultrasound signals from the patient (P), and convert the reflected ultrasound signals into corresponding electrical signals of the ultrasound signals for processing into ultrasound images;
a console (102) configured to communicate with the ultrasound probe (106), the console (102) including one or more processors (116) and a non-transitory computer-readable medium having stored thereon logic, when executed by the one or more processors (116), causes operations including:
capturing a first ultrasound image (306) of a target insertion area of a patient (P) at a first time,
capturing a second ultrasound image (406) of the target insertion area at a second time,
generating a notification indicating results of a comparison of the first ultrasound image (306) and the second ultrasound image (406), the comparison to determine whether the first ultrasound image (306) matches or substantially matches the second ultrasound image (406) such that the position of the ultrasound probe (106) when the second ultrasound image (406) was captured matches or substantially matches the position of the ultrasound probe (106) when the first ultrasound image (306) was captured, and
causing rendering of the notification.

2. The ultrasound imaging system (100) of claim 1, wherein the first time is prior to a sterilization procedure being performed to sterilize an environment surrounding the target insertion area and the second time is subsequent to the sterilization procedure.

3. The ultrasound imaging system (100) of claim 1, wherein the notification includes a comparison view display of the first ultrasound image (306) and the second ultrasound image (406) displayed in a horizontal arrangement; or
wherein the notification includes a comparison view display of the first ultrasound image (306) and the second ultrasound image (406) displayed in a vertical arrangement; or
wherein the notification incudes a comparison view display including either (i) the first ultrasound image (306) overlaid on the second ultrasound image (406), or (ii) the second ultrasound image (406) overlaid on the first ultrasound image (306).

4. The ultrasound imaging system (100) of any of the preceding claims, wherein the operations further include:
identifying one or more vessels in the first ultrasound image (306) and the second ultrasound image (406), and
providing a visual indication of the one or more vessels in the notification on a display screen (104) of the console (102).

5. The ultrasound imaging system (100) of any of the preceding claims, wherein the operations further include:
determining, through application of a trained machine learning model, whether the second ultrasound image (406) corresponds to the first ultrasound image (306) by at least a threshold amount, and
providing a visual indication of a result of applying the trained machine learning model;
optionally wherein the trained machine learning model includes a convolutional neural network.

6. A method of performing an ultrasound procedure comprising:
providing an ultrasound probe (106) including an array of ultrasonic transducers (148) configured to emit generated ultrasound signals into a patient (P), receive reflected ultrasound signals from the patient (P), and convert the reflected ultrasound signals into corresponding electrical signals of the ultrasound signals for processing into ultrasound images;
providing a console (102) configured to communicate with the ultrasound probe (106), the console (102) including one or more processors (116) and a non-transitory computer-readable medium having stored thereon logic that, when executed by the one or more processors (116), causes operations; and
instructing use of the ultrasound probe (106) and the console (102) to cause execution of the processors (116) of the console (102) to perform operations including:
capturing a first ultrasound image (306) of a target insertion area of a patient (P) at a first time,
capturing a second ultrasound image (406) of the target insertion area at a second time,
generating a notification indicating results of a comparison of the first ultrasound image (306) and the second ultrasound image (406), the comparison to determine whether the first ultrasound image (306) matches or substantially matches the second ultrasound image (406) such that the position of the ultrasound probe (106) when the second ultrasound image (406) was captured matches or substantially matches the position of the ultrasound probe (106) when the first ultrasound image (306) was captured, and
causing rendering of the notification.

7. The method of claim 6, wherein the first time is prior to a sterilization procedure being performed to sterilize an environment surrounding the target insertion area and the second time is subsequent to the sterilization procedure.

8. The method of claim 6, wherein the notification includes a comparison view display of the first ultrasound image (306) and the second ultrasound image (406) displayed in a horizontal arrangement; or
wherein the notification includes a comparison view display of the first ultrasound image (306) and the second ultrasound image (406) displayed in a vertical arrangement; or
wherein the notification incudes a comparison view display including either (i) the first ultrasound image (306) overlaid on the second ultrasound image (406), or (ii) the second ultrasound image (406) overlaid on the first ultrasound image (306).

9. The method of any of claims 6-8, wherein the operations further include:
identifying one or more vessels in the first ultrasound image (306) and the second ultrasound image (406), and
providing a visual indication of the one or more vessels in the notification on a display screen (104) of the console (102).

10. The method of any of claims 6-9, wherein the operations further include:
determining, through application of a trained machine learning model, whether the second ultrasound image (406) corresponds to the first ultrasound image (306) by at least a threshold amount, and
providing a visual indication of a result of applying the trained machine learning model.

11. A non-transitory, computer-readable medium having stored thereon logic that, when executed by one or more processors (116), causes performance of operations comprising:
capturing a first ultrasound image (306) of a target insertion area of a patient (P) at a first time,
wherein the first ultrasound image (306) is obtained by an ultrasound probe (106) including an array of ultrasonic transducers (148) configured to emit generated ultrasound signals into a patient (P), receive reflected ultrasound signals from the patient (P), and convert the reflected ultrasound signals into corresponding electrical signals of the ultrasound signals for processing into ultrasound images; and
capturing a second ultrasound image (406) of the target insertion area at a second time;
generating a notification indicating results of a comparison of the first ultrasound image (306) and the second ultrasound image (406), the comparison to determine whether the first ultrasound image (306) matches or substantially matches the second ultrasound image (406) such that the position of the ultrasound probe (106) when the second ultrasound image (406) was captured matches or substantially matches the position of the ultrasound probe (106) when the first ultrasound image (306) was captured; and
causing rendering of the notification.

12. The non-transitory, computer-readable medium of claim 11, wherein the first time is prior to a sterilization procedure being performed to sterilize an environment surrounding the target insertion area and the second time is subsequent to the sterilization procedure.

13. The non-transitory, computer-readable medium of claim 11, wherein the notification includes a comparison view display of the first ultrasound image (306) and the second ultrasound image (406) displayed in a horizontal arrangement; or
wherein the notification includes a comparison view display of the first ultrasound image (306) and the second ultrasound image (406) displayed in a vertical arrangement; or
wherein the notification incudes a comparison view display including either (i) the first ultrasound image (306) overlaid on the second ultrasound image (406), or (ii) the second ultrasound image (406) overlaid on the first ultrasound image (306).

14. The non-transitory, computer-readable medium of any of claims 11-13, wherein the operations further include:
identifying one or more vessels in the first ultrasound image (306) and the second ultrasound image (406), and
providing a visual indication of the one or more vessels in the notification on a display screen (104) of the console (102).

15. The non-transitory, computer-readable medium of any of claims 11-14, wherein the operations further include:
determining, through application of a trained machine learning model, whether the second ultrasound image (406) corresponds to the first ultrasound image (306) by at least a threshold amount, and
providing a visual indication of a result of applying the trained machine learning model;
optionally wherein the trained machine learning model includes a convolutional neural network.

## Patentansprüche

1. Ultraschallbildgebungssystem (100), umfassend:
eine Ultraschallsonde (106), die ein Array von Ultraschallwandlern (148) einschließt, die ausgebildet sind, um erzeugte Ultraschallsignale in einen Patienten (P) zu emittieren, reflektierte Ultraschallsignale vom Patienten (P) zu empfangen und die reflektierten Ultraschallsignale in entsprechende elektrische Signale der Ultraschallsignale zum Verarbeiten zu Ultraschallbildern zu konvertieren;
eine Konsole (102), die ausgebildet ist, um mit der Ultraschallsonde (106) zu kommunizieren, wobei die Konsole (102) einen oder mehrere Prozessoren (116) und ein nicht transitorisches, computerlesbares Medium aufweist, das darauf gespeicherte Logik aufweist, die, wenn sie von dem einen oder den mehreren Prozessoren (116) ausgeführt wird, Vorgänge veranlasst, einschließend:
Erfassen eines ersten Ultraschallbildes (306) eines Zieleinführungsbereichs eines Patienten (P) zu einem ersten Zeitpunkt,
Erfassen eines zweiten Ultraschallbildes (406) des Zieleinführungsbereichs zu einem zweiten Zeitpunkt,
Erzeugen einer Benachrichtigung, die Ergebnisse eines Vergleichs des ersten Ultraschallbildes (306) und des zweiten Ultraschallbildes (406) angibt, wobei der Vergleich dazu dient, zu bestimmen, ob das erste Ultraschallbild (306) mit dem zweiten Ultraschallbild (406) übereinstimmt oder im Wesentlichen übereinstimmt, so dass die Position der Ultraschallsonde (106), wenn das zweite Ultraschallbild (406) erfasst wurde, mit der Position der Ultraschallsonde (106), wenn das erste Ultraschallbild (306) erfasst wurde, übereinstimmt oder im Wesentlichen übereinstimmt, und Veranlassen eines Renderings der Benachrichtigung.

2. Ultraschallbildgebungssystem (100) nach Anspruch 1, wobei der erste Zeitpunkt vor einer Sterilisationsprozedur, die durchgeführt wird, um eine Umgebung zu sterilisieren, die den Zieleinführungsbereichs umgibt, liegt, und der zweite Zeitpunkt nach der Sterilisationsprozedur liegt.

3. Ultraschallbildgebungssystem (100) nach Anspruch 1, wobei die Benachrichtigung eine Vergleichsansichtsanzeige des ersten Ultraschallbildes (306) und des zweiten Ultraschallbildes (406), in einer horizontalen Anordnung angezeigt, einschließt; oder
wobei die Benachrichtigung eine Vergleichsansichtsanzeige des ersten Ultraschallbildes (306) und des zweiten Ultraschallbildes (406), in einer vertikalen Anordnung angezeigt, einschließt; oder
wobei die Benachrichtigung eine Vergleichsansichtsanzeige einschließt, die entweder (i) das erste Ultraschallbild (306) überlagert auf dem zweiten Ultraschallbild (406) oder (ii) das zweite Ultraschallbild (406) überlagert auf dem ersten Ultraschallbild (306) einschließt.

4. Ultraschallbildgebungssystem (100) nach einem der vorstehenden Ansprüche, wobei die Vorgänge weiter Folgendes einschließen:
Identifizieren eines oder mehrerer Gefäße im ersten Ultraschallbild (306) und im zweiten Ultraschallbild (406) und
Bereitstellen einer visuellen Angabe des einen oder der mehreren Gefäße in der Benachrichtigung auf einem Anzeigebildschirm (104) der Konsole (102).

5. Ultraschallbildgebungssystem (100) nach einem der vorstehenden Ansprüche, wobei die Vorgänge weiter Folgendes einschließen:
Bestimmen, durch Anwendung eines trainierten maschinellen Lernmodells, ob das zweite Ultraschallbild (406) dem ersten Ultraschallbild (306) um mindestens einen Schwellenwertbetrag entspricht, und
Bereitstellen einer visuellen Angabe eines Ergebnisses des Anwendens des trainierten maschinellen Lernmodells;
wobei das trainierte maschinelle Lernmodell optional ein faltungsneuronales Netzwerk einschließt.

6. Verfahren zum Durchführen einer Ultraschallprozedur, umfassend:
Bereitstellen einer Ultraschallsonde (106), die ein Array von Ultraschallwandlern (148) einschließt, die ausgebildet sind, um erzeugte Ultraschallsignale in einen Patienten (P) zu emittieren, reflektierte Ultraschallsignale vom Patienten (P) zu empfangen und die reflektierten Ultraschallsignale in entsprechende elektrische Signale der Ultraschallsignale zum Verarbeiten zu Ultraschallbildern zu konvertieren;
Bereitstellen einer Konsole (102), die ausgebildet ist, um mit der Ultraschallsonde (106) zu kommunizieren, wobei die Konsole (102) einen oder mehrere Prozessoren (116) und ein nicht transitorisches, computerlesbares Medium einschließt, das darauf gespeicherte Logik aufweist, die, wenn sie von dem einen oder den mehreren Prozessoren (116) ausgeführt wird, Operationen veranlasst; und
Anweisen einer Verwendung der Ultraschallsonde (106) und der Konsole (102), um die Ausführung der Prozessoren (116) der Konsole (102) zu veranlassen, um Vorgänge durchzuführen, die Folgendes einschließen: Erfassen eines ersten Ultraschallbildes (306) eines Zieleinführungsbereichs eines Patienten (P) zu einem ersten Zeitpunkt,
Erfassen eines zweiten Ultraschallbildes (406) des Zieleinführungsbereichs zu einem zweiten Zeitpunkt,
Erzeugen einer Benachrichtigung, die Ergebnisse eines Vergleichs des ersten Ultraschallbildes (306) und des zweiten Ultraschallbildes (406) angibt, wobei der Vergleich dazu dient, zu bestimmen, ob das erste Ultraschallbild (306) mit dem zweiten Ultraschallbild (406) übereinstimmt oder im Wesentlichen übereinstimmt, so dass die Position der Ultraschallsonde (106), wenn das zweite Ultraschallbild (406) erfasst wurde, mit der Position der Ultraschallsonde (106), wenn das erste Ultraschallbild (306) erfasst wurde, übereinstimmt oder im Wesentlichen übereinstimmt, und
Veranlassen eines Renderings der Benachrichtigung.

7. Verfahren nach Anspruch 6, wobei der erste Zeitpunkt vor einer Sterilisationsprozedur, die durchgeführt wird, um eine Umgebung zu sterilisieren, die den Zieleinführungsbereichs umgibt, liegt, und der zweite Zeitpunkt nach der Sterilisationsprozedur liegt.

8. Verfahren nach Anspruch 6, wobei die Benachrichtigung eine Vergleichsansichtsanzeige des ersten Ultraschallbildes (306) und des zweiten Ultraschallbildes (406), in einer horizontalen Anordnung angezeigt, einschließt; oder
wobei die Benachrichtigung eine Vergleichsansichtsanzeige des ersten Ultraschallbildes (306) und des zweiten Ultraschallbildes (406), in einer vertikalen Anordnung angezeigt, einschließt; oder
wobei die Benachrichtigung eine Vergleichsansichtsanzeige einschließt, die entweder (i) das erste Ultraschallbild (306) überlagert auf dem zweiten Ultraschallbild (406) oder (ii) das zweite Ultraschallbild (406) überlagert auf dem ersten Ultraschallbild (306) einschließt.

9. Verfahren nach einem der Ansprüche 6-8, wobei die Vorgänge weiter Folgendes einschließen: Identifizieren eines oder mehrerer Gefäße im ersten Ultraschallbild (306) und im zweiten Ultraschallbild (406) und Bereitstellen einer visuellen Angabe des einen oder der mehreren Gefäße in der Benachrichtigung auf einem Anzeigebildschirm (104) der Konsole (102).

10. Verfahren nach einem der Ansprüche 6-9, wobei die Vorgänge weiter Folgendes einschließen: Bestimmen durch Anwendung eines trainierten maschinellen Lernmodells, ob das
zweite Ultraschallbild (406) dem ersten Ultraschallbild (306) um mindestens einen Schwellenwertbetrag entspricht, und
Bereitstellen einer visuellen Angabe eines Ergebnisses des Anwendens des trainierten maschinellen Lernmodells.

11. Nicht transitorisches, computerlesbares Medium, das eine darauf gespeicherte Logik aufweist, die, wenn sie von einem oder mehreren Prozessoren (116) ausgeführt wird, die Durchführung von Vorgängen veranlasst, die Folgendes umfassen:
Erfassen eines ersten Ultraschallbildes (306) eines Zieleinführungsbereichs eines Patienten (P) zu einem ersten Zeitpunkt,
wobei das erste Ultraschallbild (306) durch eine Ultraschallsonde (106) erfasst wird, die ein Array von Ultraschallwandlern (148) einschließt, die ausgebildet sind, um erzeugte Ultraschallsignale in einen Patienten (P) zu emittieren und reflektierte Ultraschallsignale vom Patienten (P) zu empfangen, und die reflektierten Ultraschallsignale in entsprechende elektrische Signale der Ultraschallsignale zum Verarbeiten zu Ultraschallbildern zu konvertieren; und
Erfassen eines zweiten Ultraschallbildes (406) des Zieleinführungsbereichs zu einem zweiten Zeitpunkt;
Erzeugen einer Benachrichtigung, die die Ergebnisse eines Vergleichs des ersten Ultraschallbildes (306) und des zweiten Ultraschallbildes (406) angibt, wobei der Vergleich dazu dient, zu bestimmen, ob das erste Ultraschallbild (306) mit dem zweiten Ultraschallbild (406) übereinstimmt oder im Wesentlichen übereinstimmt, so dass die Position der Ultraschallsonde (106), wenn das zweite Ultraschallbild (406) erfasst wurde, mit der Position der Ultraschallsonde (106), wenn das erst Ultraschallbild (306) erfasst wurde, übereinstimmt oder im Wesentlichen übereinstimmt; und
Veranlassen eines Renderings der Benachrichtigung.

12. Nicht transitorisches, computerlesbares Medium nach Anspruch 11, wobei der erste Zeitpunkt vor einer Sterilisationsprozedur, die durchgeführt wird, um eine Umgebung zu sterilisieren, die den Zieleinführungsbereichs umgibt, liegt, und der zweite Zeitpunkt nach der Sterilisationsprozedur liegt.

13. Nicht transitorisches, computerlesbares Medium nach Anspruch 11, wobei die Benachrichtigung eine Vergleichsansichtsanzeige des ersten Ultraschallbildes (306) und des zweiten Ultraschallbildes (406), in einer horizontalen Anordnung angezeigt, einschließt; oder
wobei die Benachrichtigung eine Vergleichsansichtsanzeige des ersten Ultraschallbildes (306) und des zweiten Ultraschallbildes (406), in einer vertikalen Anordnung angezeigt, einschließt; oder
wobei die Benachrichtigung eine Vergleichsansichtsanzeige einschließt, die entweder (i) das erste Ultraschallbild (306) überlagert auf dem zweiten Ultraschallbild (406) oder (ii) das zweite Ultraschallbild (406) überlagert auf dem ersten Ultraschallbild (306) einschließt.

14. Nicht transitorisches, computerlesbares Medium nach einem der Ansprüche 11-13, wobei die Vorgänge weiter Folgendes einschließen:
Identifizieren eines oder mehrerer Gefäße im ersten Ultraschallbild (306) und im zweiten Ultraschallbild (406) und
Bereitstellen einer visuellen Angabe des einen oder der mehreren Gefäße in der Benachrichtigung auf einem Anzeigebildschirm (104) der Konsole (102).

15. Nicht transitorisches, computerlesbares Medium nach einem der Ansprüche 11-14, wobei die Vorgänge weiter Folgendes einschließen:
Bestimmen, durch Anwendung eines trainierten maschinellen Lernmodells, ob das zweite Ultraschallbild (406) dem ersten Ultraschallbild (306) um mindestens einen Schwellenwertbetrag entspricht, und
Bereitstellen einer visuellen Angabe eines Ergebnisses des Anwendens des trainierten maschinellen Lernmodells;
wobei das trainierte maschinelle Lernmodell optional ein faltungsneuronales Netzwerk einschließt.

## Revendications

1. Système d'imagerie ultrasonore (100), comprenant :
une sonde ultrasonore (106) incluant un réseau de transducteurs ultrasonores (148) configurés pour émettre des signaux ultrasonores générés dans un patient (P), recevoir des signaux ultrasonores réfléchis provenant du patient (P) et convertir les signaux ultrasonores réfléchis en signaux électriques correspondants des signaux ultrasonores pour traitement en images ultrasonores ;
une console (102) configurée pour communiquer avec la sonde ultrasonore (106), la console (102) incluant un ou plusieurs processeurs (116) et un support non transitoire lisible par ordinateur présentant une logique stockée sur celui-ci qui, lorsqu'elle est exécutée par les un ou plusieurs processeurs (116), provoque des opérations incluant :
la capture d'une première image ultrasonore (306) d'une zone d'insertion cible d'un patient (P) à un premier moment,
la capture d'une seconde image ultrasonore (406) de la zone d'insertion cible à un second moment,
la génération d'une notification indiquant des résultats d'une comparaison de la première image ultrasonore (306) et de la seconde image ultrasonore (406), la comparaison servant à déterminer si la première image ultrasonore (306) correspond ou correspond sensiblement à la seconde image ultrasonore (406) de sorte que la position de la sonde ultrasonore (106) lors de la capture de la seconde image ultrasonore (406) corresponde ou corresponde sensiblement à la position de la sonde ultrasonore (106) lors de la capture de la première image ultrasonore (306), et le fait de provoquer l'affichage de la notification.

2. Système d'imagerie ultrasonore (100) selon la revendication 1, dans lequel le premier moment est antérieur à une opération de stérilisation étant effectuée pour stériliser un environnement entourant la zone d'insertion cible et le second moment est postérieur à l'opération de stérilisation.

3. Système d'imagerie ultrasonore (100) selon la revendication 1, dans lequel la notification inclut un affichage de vue comparative de la première image ultrasonore (306) et de la seconde image ultrasonore (406) affichées à l'horizontale ; ou
dans lequel la notification inclut un affichage de vue comparative de la première image ultrasonore (306) et de la seconde image ultrasonore (406) affichées à la verticale ; ou,
dans lequel la notification inclut un affichage de vue comparative incluant soit (i) la première image ultrasonore (306) superposée à la seconde image ultrasonore (406), soit (ii) la seconde image ultrasonore (406) superposée à la première image ultrasonore (306).

4. Système d'imagerie ultrasonore (100) selon l'une quelconque des revendications précédentes, dans lequel les opérations incluent en outre :
l'identification d'un ou plusieurs vaisseaux dans la première image ultrasonore (306) et la seconde image ultrasonore (406), et
la fourniture d'une indication visuelle des un ou plusieurs vaisseaux dans la notification sur un écran d'affichage (104) de la console (102).

5. Système d'imagerie ultrasonore (100) selon l'une quelconque des revendications précédentes, dans lequel les opérations incluent en outre :
la détermination, par application d'un modèle d'apprentissage automatique entraîné, pour établir si la seconde image ultrasonore (406) correspond à la première image ultrasonore (306) du moins dans une mesure seuil, et
la fourniture d'une indication visuelle d'un résultat de l'application du modèle d'apprentissage automatique entraîné ;
dans lequel, éventuellement, le modèle d'apprentissage automatique entraîné inclut un réseau neuronal convolutif.

6. Procédé d'exécution d'une intervention aux ultrasons comprenant :
la fourniture d'une sonde ultrasonore (106) incluant un réseau de transducteurs ultrasonores (148) configurés pour émettre des signaux ultrasonores générés dans un patient (P), recevoir des signaux ultrasonores réfléchis provenant du patient (P) et convertir les signaux ultrasonores réfléchis en signaux électriques correspondants des signaux ultrasonores pour traitement en images ultrasonores ;
la fourniture d'une console (102) configurée pour communiquer avec la sonde ultrasonore (106), la console (102) incluant un ou plusieurs processeurs (116) et un support non transitoire lisible par ordinateur présentant une logique stockée sur celui-ci qui, lorsqu'elle est exécutée par les un ou plusieurs processeurs (116), provoque des opérations ; et
l'ordre donné d'utiliser la sonde ultrasonore (106) et la console (102) pour provoquer l'exécution des processeurs (116) de la console (102) afin d'effectuer des opérations incluant : la capture d'une première image ultrasonore (306) d'une zone d'insertion cible d'un patient (P) à un premier moment,
la capture d'une seconde image ultrasonore (406) de la zone d'insertion cible à un second moment,
la génération d'une notification indiquant des résultats d'une comparaison de la première image ultrasonore (306) et de la seconde image ultrasonore (406), la comparaison servant à déterminer si la première image ultrasonore (306) correspond ou correspond sensiblement à la seconde image ultrasonore (406) de sorte que la position de la sonde ultrasonore (106) lors de la capture de la seconde image ultrasonore (406) corresponde ou corresponde sensiblement à la position de la sonde ultrasonore (106) lors de la capture de la première image ultrasonore (306), et
le fait de provoquer l'affichage de la notification.

7. Procédé selon la revendication 6, dans lequel le premier moment est antérieur à une opération de stérilisation étant effectuée pour stériliser un environnement entourant la zone d'insertion cible et le second moment est postérieur à l'opération de stérilisation.

8. Procédé selon la revendication 6, dans lequel la notification inclut un affichage de vue comparative de la première image ultrasonore (306) et de la seconde image ultrasonore (406) affichées à l'horizontale ; ou
dans lequel la notification inclut un affichage de vue comparative de la première image ultrasonore (306) et de la seconde image ultrasonore (406) affichées à la verticale ; ou,
dans lequel la notification inclut un affichage de vue comparative incluant soit (i) la première image ultrasonore (306) superposée à la seconde image ultrasonore (406), soit (ii) la seconde image ultrasonore (406) superposée à la première image ultrasonore (306).

9. Procédé selon l'une quelconque des revendications 6 à 8, dans lequel les opérations incluent en outre : l'identification d'un ou plusieurs vaisseaux dans la première image ultrasonore (306) et la seconde image ultrasonore (406), et la fourniture d'une indication visuelle des un ou plusieurs vaisseaux dans la notification sur un écran d'affichage (104) de la console (102).

10. Procédé selon l'une quelconque des revendications 6 à 9, dans lequel les opérations incluent en outre : la détermination, par application d'un modèle d'apprentissage automatique entraîné, pour établir si la
seconde image ultrasonore (406) correspond à la première image ultrasonore (306) du moins dans une mesure seuil, et
la fourniture d'une indication visuelle d'un résultat de l'application du modèle d'apprentissage automatique entraîné.

11. Support non transitoire lisible par ordinateur présentant une logique stockée sur celui-ci qui, lorsqu'elle est exécutée par un ou plusieurs processeurs (116), provoque l'exécution d'opérations comprenant :
la capture d'une première image ultrasonore (306) d'une zone d'insertion cible d'un patient (P) à un premier moment,
dans lequel la première image ultrasonore (306) est obtenue par une sonde ultrasonore (106) incluant un réseau de transducteurs ultrasonores (148) configurés pour émettre des signaux ultrasonores générés dans un patient (P), recevoir des signaux ultrasonores réfléchis provenant du patient (P) et convertir les signaux ultrasonores réfléchis en signaux électriques correspondants des signaux ultrasonores pour traitement en images ultrasonores ; et
la capture d'une seconde image ultrasonore (406) de la zone d'insertion cible à un second moment ;
la génération d'une notification indiquant des résultats d'une comparaison de la première image ultrasonore (306) et de la seconde image ultrasonore (406), la comparaison servant à déterminer si la première image ultrasonore (306) correspond ou correspond sensiblement à la seconde image ultrasonore (406) de sorte que la position de la sonde ultrasonore (106) lors de la capture de la seconde image ultrasonore (406) corresponde ou corresponde sensiblement à la position de la sonde ultrasonore (106) lors de la capture de la première image ultrasonore (306) ; et
le fait de provoquer l'affichage de la notification.

12. Support non transitoire lisible par ordinateur selon la revendication 11, dans lequel le premier moment est antérieur à une opération de stérilisation étant effectuée pour stériliser un environnement entourant la zone d'insertion cible et le second moment est postérieur à l'opération de stérilisation.

13. Support non transitoire lisible par ordinateur selon la revendication 11, dans lequel la notification inclut un affichage de vue comparative de la première image ultrasonore (306) et de la seconde image ultrasonore (406) affichées à l'horizontale ; ou
dans lequel la notification inclut un affichage de vue comparative de la première image ultrasonore (306) et de la seconde image ultrasonore (406) affichées à la verticale ; ou,
dans lequel la notification inclut un affichage de vue comparative incluant soit (i) la première image ultrasonore (306) superposée à la seconde image ultrasonore (406), soit (ii) la seconde image ultrasonore (406) superposée à la première image ultrasonore (306).

14. Support non transitoire lisible par ordinateur selon l'une quelconque des revendications 11 à 13, dans lequel les opérations incluent en outre :
l'identification d'un ou plusieurs vaisseaux dans la première image ultrasonore (306) et la seconde image ultrasonore (406), et
la fourniture d'une indication visuelle des un ou plusieurs vaisseaux dans la notification sur un écran d'affichage (104) de la console (102).

15. Support non transitoire lisible par ordinateur selon l'une quelconque des revendications 11 à 14, dans lequel les opérations incluent en outre :
la détermination, par application d'un modèle d'apprentissage automatique entraîné, pour établir si la seconde image ultrasonore (406) correspond à la première image ultrasonore (306) du moins dans une mesure seuil, et
la fourniture d'une indication visuelle d'un résultat de l'application du modèle d'apprentissage automatique entraîné ;
dans lequel, éventuellement, le modèle d'apprentissage automatique entraîné inclut un réseau neuronal convolutif.
